# EUROPEAN PATENT SPECIFICATION

(11) **EP 3 863 534 B1**
(45) Date of publication and mention of the grant of the patent: **13.03.2024**
(21) Application number: 19795390.4
(22) Date of filing: 09.10.2019
(51) Int. Cl.: A61N 1/372, A61B 17/221, A61N 1/375, A61B 17/22, A61N 1/05, A61B 17/00

(54) **MEDICAL DEVICE RETRIEVAL TOOL WITH ADJUSTABLE CURVE**
RÜCKHOLWERKZEUG FÜR MEDIZINISCHE VORRICHTUNGEN MIT EINSTELLBARER KRÜMMUNG
OUTIL DE RÉCUPÉRATION DE DISPOSITIF MÉDICAL À COURBE RÉGLABLE

(30) Priority: 10.10.2018 US 201862743939 P; 08.10.2019 US 201916596252
(43) Date of publication of application: 18.08.2021
(73) Proprietor: Medtronic, Inc., Minneapolis, Minnesota 55432 (US)
(72) Inventor: DRAKE, Ronald A., Minneapolis, Minnesota 55432 (US); LANGE, Kendra, Minneapolis, Minnesota 55432 (US); HILPISCH, Kathryn, Minneapolis, Minnesota 55432 (US); EGGEN, Michael D., Minneapolis, Minnesota 55432 (US); BONNER, Matthew D., Minneapolis, Minnesota 55432 (US); COLIN, Brian P., Minneapolis, Minnesota 55432 (US); VATTEROTT, Pierce J., Minneapolis, Minnesota 55432 (US)
(74) Representative: Zimmermann & Partner Patentanwälte mbB
(86) International application number: PCT/US2019/055363
(87) International publication number: WO 2020/076926

(56) References cited:
- WO-A1-2017/058315
- US-A1- 2003 028 173
- US-A1- 2013 211 415
- US-A1- 2017 043 158
- US-B2- 9 526 522

## Description

### TECHNICAL FIELD

This disclosure relates to interventional medical systems for retrieving medical devices, such as from implant sites within a patient.

### BACKGROUND

In some examples, implantable cardiac pacemakers include a pulse generator device to which one or more flexible elongate lead wires are coupled. The pulse generator device may be implanted in a subcutaneous pocket, remote from the heart, and each of the one or more lead wires extends therefrom to a corresponding electrode, coupled thereto and positioned at a pacing site, either endocardial or epicardial. Mechanical and/or MRI compatibility issues may be associated with elongate lead wires. Relatively compact implantable medical device (IMD) have been developed that are wholly contained within a relatively compact package, the entirety of which is configured for implant in close proximity to the pacing site, US 2017/0043158 A1 relates to interventional medical systems and catheters.

### SUMMARY

The invention is defined by the independent claim 1. The present disclosure describes example medical systems and techniques for retrieving an implantable medical device (IMD), such as a relatively compact IMD. In some examples, an IMD retrieval system may include an elongate outer tubular member defining longitudinally extending lumen terminating in a distal cup and a retrieval assembly position in the lumen of the elongate outer tubular member. The retrieval assembly may include a retrieval member having a distal snare moveable between an expanded configuration to receive the IMD and a contracted to engage the IMD and a fixed-curve catheter The fixed-curve catheter may define a lumen in which the retrieval assembly extends in sliding engagement, and the fixed-curve catheter may extend in sliding engagement within the lumen of the elongate outer tubular member. The fixed-curve catheter may include a resilient material forming a distal curve relative to a longitudinal axis of the fixed-curve catheter. The distal curve of the fixed-curve catheter may be controllable between a substantially straight configuration when the distal curve is retracted into or otherwise located within the distal cup of the elongate outer tubular member and an at least partially curved configuration when the distal curve is extended from the distal end of the distal cup. In this way, the fixed-curve catheter provides a clinician with improved control of an orientation of the snare, distal cup, and other portions of the IMD retrieval system to reduce retrieval time.

In some examples, an implantable medical device (IMD) retrieval system may include an elongate outer tubular member and a retrieval assembly. The elongate outer tubular member may include a handle configured to control an orientation of the elongate outer tubular member, and a longitudinally extending lumen terminating in a distal cup. The distal cup may be configured to receive the IMD. The retrieval assembly may include a retrieval member and a fixed-curve catheter. The retrieval member may include an elongate member terminating in at least one snare, where the at least one snare is moveable between an expanded configuration and a contracted configuration; and a torque member configured to control rotation of the retrieval member about a longitudinal axis of the elongate member. The fixed-curve catheter may include a core layer comprising a resilient material and defining a lumen in which the retrieval assembly extends in sliding engagement. The fixed-curve catheter may define a distal curve and may extend in sliding engagement within the lumen of the elongate outer tubular member.

In some non-claimcd examples, a method of retrieving an implantable medical device may include introducing an implantable medical device (IMD) retrieval system into the vasculature of a patient The IMD retrieval system may include an elongate outer tubular member including a longitudinally extending lumen terminating in a distal cup, where the distal cup is configured to receive the IMD, and a retrieval assembly including a retrieval member that includes an elongate member terminating in at least one snare, where the at least one snare is moveable between an expanded configuration and a contracted configuration, and a fixed-curve catheter that includes a resilient material forming a distal curve and defining a lumen in which the retrieval assembly extends in sliding engagement where the fixed-curve catheter extends in sliding engagement within the lumen of the elongate outer tubular member. The method also may include controlling the retrieval assembly to receive the IMD in the snare. The method also may include controlling the retrieval assembly to engage the IMD in the snare. The method also may include controlling the retrieval assembly to retrieve the IMD.

The details of one or more examples are set forth in the accompanying drawings and the description below. Other features, objects, and advantages will be apparent from the description and drawings, and from the claims

### BRIEF DESCRIPTION OF THE DRAWINGS

FIG. 1 is a conceptual and schematic diagram illustrating potential cardiac implant sites for a relatively compact IMD.
FIG. 2 is a conceptual and schematic diagram illustrating an example relatively compact IMD having been delivered from a catheter to an implant site.
FIG. 3 is a plan view illustrating an example relatively compact IMD.
FIGS. 4A-4E are conceptual and schematic diagrams illustrating an example IMD retrieval system.
FIG. 5A-5D are conceptual diagrams illustrating an example IMD retrieval system.
FIGS. 6A and 6B are conceptual diagrams illustrating cross-sectional side and axial views of an example IMD retrieval system.
FIGS. 7A-7E are conceptual diagrams illustrating adjustable control of the degree of curve of an example IMD retrieval system.
FIGS. 8A-8H are conceptual diagrams illustrating engagement of the snare of an IMD retrieval system with the proximal retrieval feature on an IMD.
FIGS. 9A-9F are conceptual diagrams illustrating retrieval of an implantable medical device by urging the distal end of a fixed-curve catheter against the proximal retrieval feature of an IMD to facilitate seating of the IMD in the distal cup of an IMD retrieval system.
FIGS. 10A and 10B are conceptual diagrams illustrating retrieval of an IMD by using a fixed-curved catheter of an IMD retrieval system to cinch a snare into a contracted configuration.
FIG. 11 is a flow diagram illustrating an example method of manufacturing an implantable medical device retrieval system, which method is not claimed but useful for understanding the invention.
FIG. 12 is a flow diagram illustrating an example method or using an implantable medical device retrieval system, which method is not claimed but useful for understanding the invention.

### DETAILED DESCRIPTION

The disclosure describes systems and techniques for retrieving an implantable medical device (IMD). An example system includes an elongate outer tubular member and a retrieval assembly. The elongate outer tubular member may include a longitudinally extending lumen terminating in a distal cup. The distal cup may be configured to receive the IMD. For example, an inner diameter of the distal cup in proximity to a distal opening of the lumen may be sized to contain the IMD therein.

The retrieval assembly may include a retrieval member and a fixed-curve catheter. The retrieval member may include an elongate member terminating in at least one snare. The at least one snare may be moveable between an expanded configuration and a contracted configuration. For example, the expanded configuration may be configured to receive the IMD and the contracted configuration may be configured to engage the IMD to attach the retrieval member to the IMD via the snare for retrieval of the IMD. The fixed-curve catheter may include a resilient material forming a distal curve. The distal curve is relative to a longitudinal axis of the fixed-curve catheter. The fixed-curve catheter also may define a lumen in which the retrieval assembly extends in sliding engagement. Further, the fixed-curve catheter may extend in sliding engagement within the lumen of the outer tubular member.

As will be described further below, the curved portion of the fixed-curved catheter may be selectively straightened or bent as desired to a retrieval process, e.g., by sliding the curved portion of the fixed curve catheter relative to the distal cup of the elongate outer tubular member. For example, when the curved portion of the fixed-curved catheter is slid to extend all the way out of the distal cup, the curved portion of the fixed-curved catheter may have exhibit a first curvature, e.g., the fixed-curve. As the curved portion of the fixed-curved catheter is slid into the rigid cup, the rigid cup may apply a force to the curved portion based on the generally straight configuration of die rigid cup and rigid material. The curvature of the curved portion of the fixed-curved catheter may decrease the further the curved portion is slid within the straight rigid cup. When the curved portion of the fixed-curved catheter is entirely within the straight rigid cup, the straight rigid cup may act on the curved portion such that the curved portion has a substantially straight orientation or at least a curvature that is less than the first curvature of the curved portion when it is entirely outside the straight rigid cup.

In some examples, an IMD may be difficult to retrieve after implantation. For example, retrieval of an IMD implanted in a chamber of the heart of a patient may include navigating an IMD retrieval system including a catheter through vasculature to an implant site. In some examples, an IMD retrieval system may include a snare, such as a loop snare, extending through a lumen of the catheter. A clinician may manipulate the orientation of the snare to receive the IMD (e.g., position a loop snare over the IMD), engage the IMD (e.g., tighten the loop snare to mechanically couple to the IMD), and/or retrieve the IMD (e.g., pull the IMD from the implant tissue). However, manipulation of the snare may be limited to controlling longitudinal position or rotation of the snare relative to a longitudinal axis of the catheter. For example, once positioned near the implant site, the catheter may be difficult to orient in a three dimensional space.

To improve manipulation of the snare, the disclosure describes a system for retrieving an IMD that includes an elongate outer tubular member and a retrieval assembly. The elongate outer tubular member may include a longitudinally extending lumen terminating in a distal cup that is configured to receive the IMD. In some examples, a distal portion of the elongate outer tubular member, e.g., proximal the distal cup, may include a distal curve relative to the longitudinal axis of the elongate outer tubular member.

The retrieval assembly includes a retrieval member and a fixed-curve catheter. The retrieval member includes an elongate member terminating in at least one snare. The fixed-curve catheter includes a resilient material forming a distal curve. The distal curve is relative to a longitudinal axis of die fixed-curve catheter. The fixed-curve catheter may extend in sliding engagement within the lumen of the outer tubular member. By including the distal curve in sliding engagement with the lumen of the outer tubular member, the fixed-curve catheter is controllable between a substantially straight configuration when retracted into the distal cup of the outer tubular member and an at least partially curved configuration when extended from the distal end of the distal cup. In other words, the distal cup allows the fixed-curve catheter to take on an adjustable curve shape depending on the longitudinal position of the distal curve of the fixed-curve catheter relative to the distal opening of the distal cup.

For example, when the distal curve of the fixed-curve catheter remains inside the distal cup, a longitudinal axis of the retrieval member may be substantially the same as the longitudinal axis of the elongate outer tubular member. In other words, the distal cup urges the fixed-curve catheter into a substantially straight configuration, e.g., straight or nearly straight relative to the longitudinal axis of the elongate outer tubular member. When die distal curve of the fixed-curve catheter is at least partially extended distal to the distal opening of the distal cup, a longitudinal axis of the retrieval member may bend at an angle relative to the longitudinal axis of the elongate outer tubular member. In other words, the fixed-curve catheter is allowed to take on its shape as the distal curve extends out of the distal cup. If only a portion of the distal curve extends out of the distal cup, the curve may be slight. If more of the distal curve extends out of the distal cup, the curve may be more pronounced.

The adjustable curve of the fixed-curve catheter provides enhanced steering of the snare. For example, by enabling a clinician to control a degree of curvature of the fixed-curve catheter. In examples in which the elongate outer tubular member is curved, the fixed-curve catheter may enable secondary curves. The secondary curves may be manipulated by a clinician, e.g., by controlling the degree of curvature of die fixed-curve catheter, the radial orientation of the distal curve of the fixed-curve catheter relative to the curve of the elongate outer tubular member, or both, to improve control of the snare in three dimensional space. By providing greater control of the snare in a three-dimensional space, the retrieval system may reduce the time to properly receive the IMD for engagement, improve navigation of the snare in smaller spaces, and/or maintain tension transverse to a longitudinal axis of the IMD during engagement.

In some examples, when the snare receives the IMD (e.g., goes over the housing of the IMD), the clinician may control the degree and/or orientation of the distal curve fixed-curve catheter to apply a side load to the IMD housing, e.g., tipping the IMD slightly. Application of a side load to the IMD housing may be useful in forcing one side of the snare to ride along the IMD housing as the snare is controlled from an expanded configuration to a contracted configuration. As the snare is slid proximally over the IMD housing, the side load helps the snare engage a groove in a proximal retrieval feature on the IMD. By facilitating engagement with the IMD, the system may reduce the time to properly engage the IMD for retrieval.

In some examples, once the snare has engaged the IMD, e.g., via the proximal retrieval feature, the orientation of the IMD relative to the distal cup may make it difficult to seat the distal cup over the IMD prior to retrieval. For example, the distal opening of the distal cup may catch in the groove of the proximal retrieval feature. The fixed-curve catheter may facilitate reorienting the distal cup by rotating the distal cup relative to the fixed-curve catheter, and thereby relative to the IMD. In other words, spinning the fixed-curve catheter allows the distal cup to change orientation relative to the IMD, guiding the distal cup to an orientation that allows the IMD to be retrieved into the distal cup. Additionally, or alternatively, once the snare has engaged the IMD, e.g., the proximal retrieval feature, the fixed-curve catheter may be slid up to the proximal retrieval feature. The tip of the fixed-curve catheter may include a coil tip (or similar) that is sized to divert the distal cup away from the groove of the proximal retrieval feature. In other words, with the coil tip of die fixed-curve catheter pressed into the IMD, die groove of the proximal retrieval feature is covered, and the distal cup is prevented from catching on the groove.

In some examples, the system may be used for lead extraction with the elongate outer tubular member providing countertraction. Countertraction may be desirable during lead extraction to prevent the heart wall from inverting or pulling with the lead as the lead is pulled. For example, if the lead is encapsulated, it will not pull out (or rotate out) easily. Applying countertraction provides a counter force to focus the extraction energy to the lead implant site.

In some examples, the fixed-curved catheter may be used as part of the snare cinching mechanism. For example, a snare including a metal loop element may be locked in place relative to an elongate member, e.g., a snare catheter, thereby engaging the IMD. Then the snare catheter may be slid as a unit inside the fixed-curved catheter, e.g., such that a distal tip of the fixed-curve catheter contacts the engaged snare and IDM. The snare catheter may be held relative to the fixed-curve catheter via a torque member to keep the snare tight on the proximal retrieval feature during removal. In some examples, sliding the snare catheter inside the fixed-curve catheter may block a groove of a proximal retrieval feature of an IMD to prevent the proximal retrieval feature from catching on the distal cup. In some examples, sliding the snare catheter inside the fixed-curve catheter may be used to orient the IMD to align with the distal cup. In some examples, sliding the snare catheter inside the fixed-curve catheter may enable the fixed-curve catheter to guide the distal cup over the IMD. In some examples, the fixed curve catheter can be used in place of retrieval member (e.g., snare and snare catheter) to minimize steps.

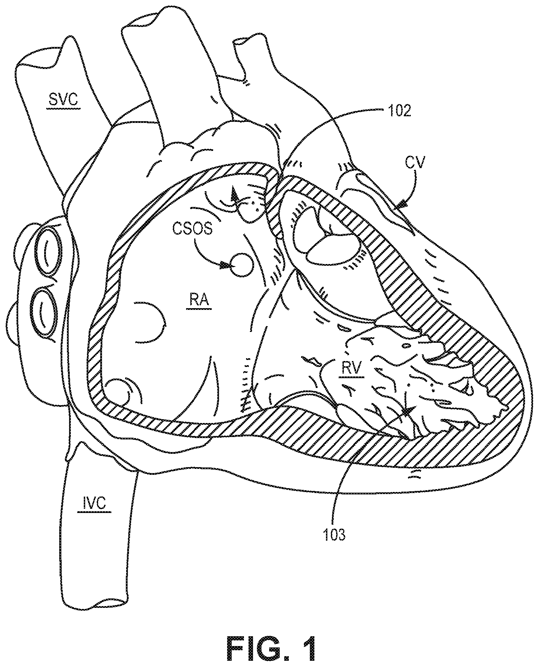

FIG. 1 is a conceptual and schematic diagram illustrating potential cardiac implant sites for a relatively compact IMD. For example, a relatively compact IMD may be implanted on or within heart 100 of a patient, such as within an appendage 102 of a right atrium (RA), within a coronary vein (CV) via a coronary sinus ostium (CSOS), or in proximity to an apex 103 of a right ventricle (RV). In other examples, the relatively compact IMD may be implanted on other portions of heart 1 00 or implanted in locations other than heart 100, such as any suitable implant site in a body of the patient.

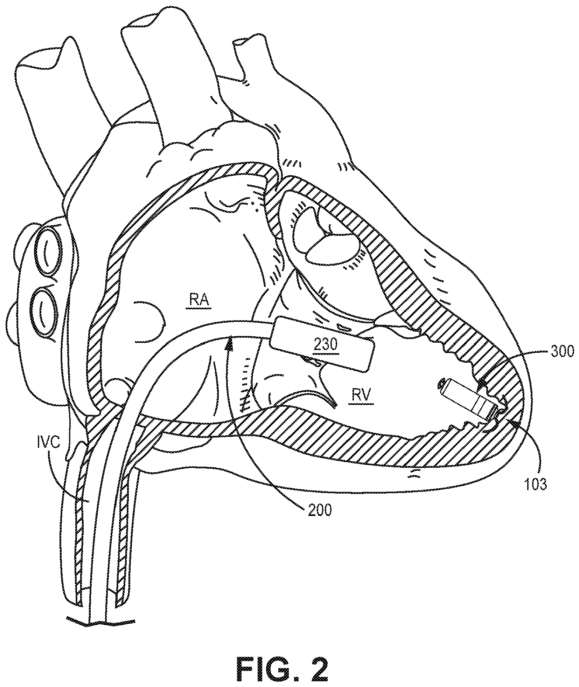

The relatively compact implantable medical device may be implanted using a cardiac catheter system. FIG. 2 is a conceptual and schematic diagram illustrating an example relatively compact implantable medical device 300 (IMD 300) having been implanted by a clinician using a IMD implant system 200. In some examples, IMD implant system 200 may implant IMD 300 using the techniques and/or interventional medicine systems, tools, or assemblies as described in United States Patent No. 9,526,522.

For example, a clinician may advance IMD implant system 200 into the RV of heart 100 through the RA. and inferior vena cava (IVC) via a femoral access site. The clinician may deploy IMD 300 from a device receptacle 230 of IMD implant system 200.

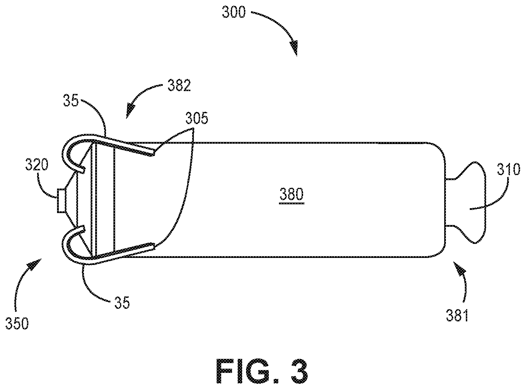

FIG. 3 is a plan view of implantable medical device 300. FIG. 3 illustrates device 300 including a housing 380 extending from a proximal end 381 thereof to a distal end 382 In some examples, housing 380 may be hermetically sealed. In some examples, an electronic controller (not shown), for example, a pulse generator and an associated power supply, may be contained in housing 380. Device 300 includes an electrode 320 and a fixation member 350, both mounted in proximity to distal end 382 of housing 380. Electrode 320 may be electrically coupled to the controller via a hermetically sealed feedthrough assembly (not shown). Housing 380 may be formed from a biocompatible and biostable metal such as, for example, titanium, and overlaid with an insulative layer such as, for example, medical grade polyurethane, parylene, or silicone. In some examples, device 300 may include another electrode (not shown), for example, formed by removing a portion of the insulative layer to expose the metallic surface of housing 380. The other electrode may function in conjunction with electrode 320 for bipolar pacing and sensing, when fixation member 350 secures electrode 320 in intimate tissue contact at a target implant site. FIG. 3 further illustrates device 300 including an optional proximal retrieval feature 310 joined to proximal end 381 of housing 380. Proximal retrieval feature 310 may be configured for snaring, for example, by a retrieval member.

With further reference to FIG. 3. device fixation member 350 includes a plurality of fingers 35 spaced apart from one another around a perimeter of device housing distal end 382, wherein fingers 35 are configured to fix device 300 to tissue at an implant site. Although only two fingers 35 of fixation member 350 are illustrated in FIG 3, fixation member 350 may include as many as eight fingers 35 or more than eight fingers 35. According to one example, fixation fingers 35 are integrally formed with one another, having been cut from nickel titanium alloy tubing or other biocompatible tubing. After cutting the nickel titanium alloy tubing, fingers 35 may be shaped by bending and holding fingers 35 in the illustrated curvature while undergoing heat treatment. Fixation member 350 may be mounted to distal end 382 of device housing 380, for example, in a manner similar to that described for a fixation component 102 in United States Patent Application Publication No. 2012/0172690. The super-elastic nature of nickel titanium alloy allows fingers 35 to elastically deform between a relaxed condition and an extended condition, in which a free end 305 of each finger extends distally away from distal end 382 of device housing 380.

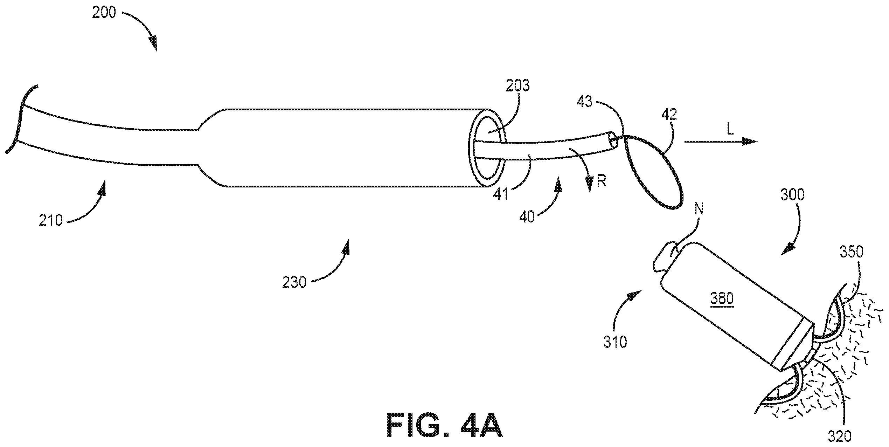

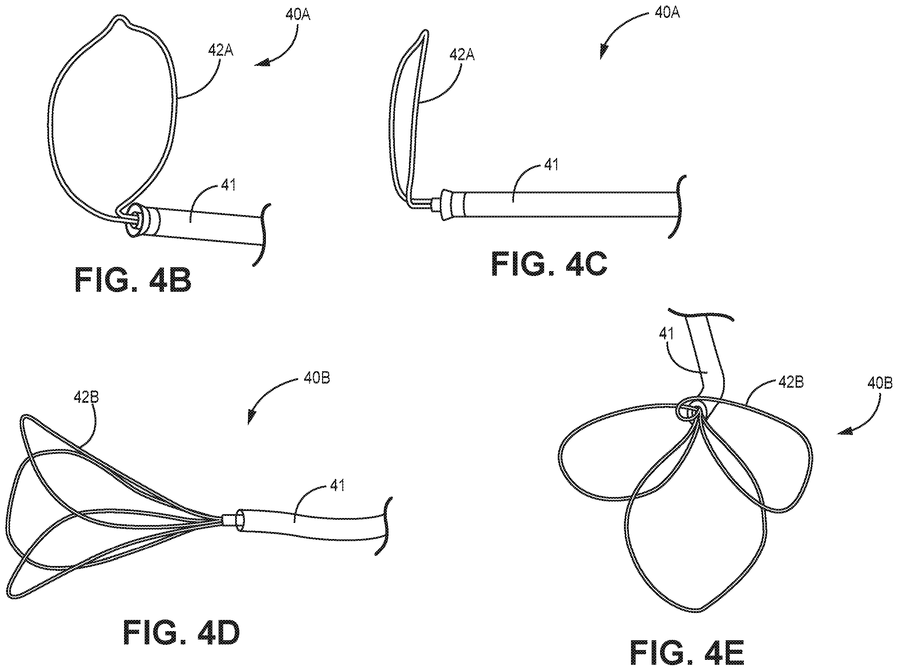

In some examples, it may be necessary to retrieve the implanted device 300 from an implant site FIG. 4A--4E are conceptual and schematic diagrams illustrating an example implantable medical device retrieval system. FIG. 4A illustrates retrieval system 200 having been advanced to the implant site. Retrieval system 200 includes elongate outer tubular member 210 including distal cup 230 and a retrieval assembly 40. Retrieval assembly 40 has been extended through a distal opening 203 of distal cup 230. In some examples, distal cup 230 may include a substantially rigid material, e.g., relatively more rigid than elongate outer tubular member 210 and/or retrieval assembly 40. Retrieval assembly 40 includes an elongate member 43 extending within a catheter 4 land defining a distal snare 42. Snare 42 may be slidably engaged within catheter 41 to expand (e.g., open) and contract (e.g., close) a loop thereof. In some examples, such as illustrated in FIGS. 4B and 4C, retrieval assembly 40A may include a loop snare 42A. Loop snare 42A includes a single metal wire loop. In some examples, such as illustrated in FIGS. 4D and 4E, retrieval assembly 40B may include a triple loop snare 42B. Triple loop snare 42B may include three metal wire loops. In other examples, snare 42 may include other suitable types of snare, including, but not limited to, a net or a hook. Regardless of the type of snare, snare 42 may include any suitable braided or non-braided metal, such as, for example, stainless steel, nickel titanium alloy, radiopaque elements such as gold or platinum iridium coil wrapping wires and/or bands, or the like.

FIG. 4A illustrates snare 42 is in an expanded configuration. A clinician may maneuver the snare 42 into position around a neck N of proximal retrieval feature 310. For example, a clinician may attempt to deflect catheter 41 in the direction of arrow R by rotating catheter 41 relative to elongate outer tubular member 210. In some examples, a proximal end of retrieval assembly 40 may include a torque member to rotate catheter 41. Additionally, or alternatively, the clinician may move catheter in the direction of arrow L by extending catheter 41 distally relative to elongate outer tubular member 210. In some examples, maneuvering retrieval assembly 40 in only directions R and L may be difficult, particularly in view of imaging the movement of retrieval system 200 via fluoroscopy, and time consuming. For example, due to the limited directional control of snare 42, the clinician may have difficulty orienting the snare in three-dimensional scape to revise IMD 300. Additionally, or alternatively, the clinician may inadvertently engage snare 42 around device housing 380, rather than around neck N of proximal retrieval feature 310. Additionally, or alternatively, once the clinician has successfully engaged snare 42 around proximal retrieval feature neck N, IMD 300 may be misaligned with distal opening 203 of receptacle 230, such that extracting device 300 from the implant site and drawing device 300 into receptacle 230 may be difficult.

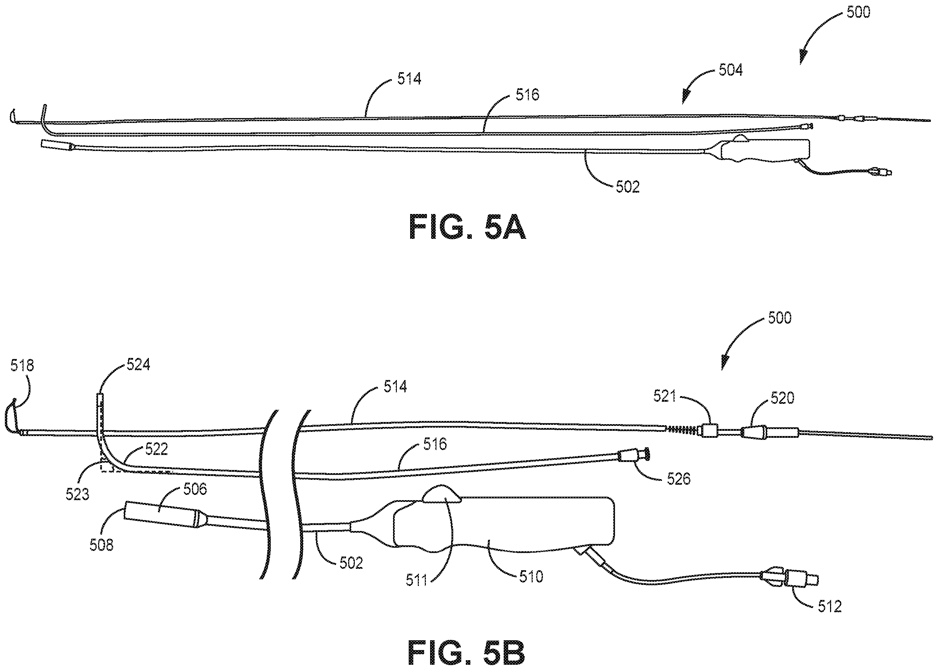

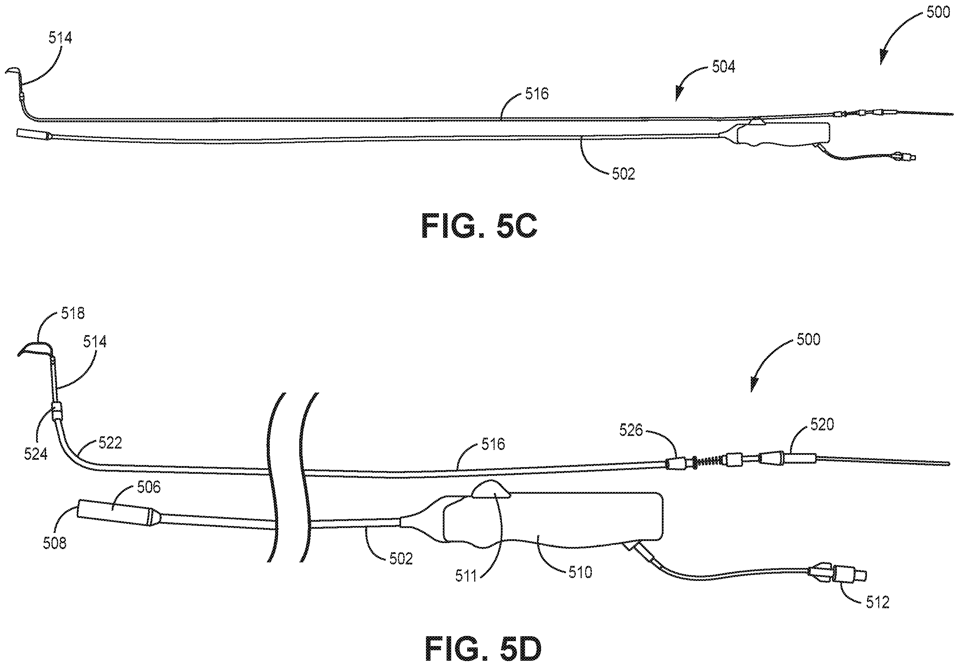

In some examples, a fixed-curved catheter may be used to improve control of the orientation of retrieval system 200, such as snare 42. FIG. 5A-5D are conceptual diagrams illustrating an example IMD retrieval system 500. IMD retrieval system 500 includes elongate outer tubular member 502 and retrieval assembly 504. Outer tubular member 502 may include a longitudinally extending lumen terminating in distal tip 508 of a distal cup 506. Distal cup 506 may be configured to receive an IMD, such as IMD 300. For example, distal cup 506 may include one or more walls defining a cavity having an inner diameter sized to contain an IMD therein. In some examples, distal cup 506 may be substantially straight, e.g., the walls of distal cup 506 may be substantially parallel to a longitudinal axis defined by the outer tubular member 502. In some examples, distal cup 506 may include a substantially rigid material, e.g., relatively more rigid than elongate outer tubular member 502 and/or retrieval assembly 504. For example, distal cup 506 may be sufficiently rigid to provide a selective resistive force against retrieval assembly 504 (e.g., distal curve 522 of fixed-curve catheter 516) to urge retrieval assembly 504 into a substantially straight configuration when retracted into distal cup 506. In some examples, outer tubular member 502 may include a proximal handle 510 configured to enable a clinician to manipulate outer tubular member 502. For example, a clinician may use handle 510 to introduce outer tubular member 502 into the vasculature of a patient, rotate outer tubular member 502, and/or introduce therapeutic agents via coupling 512. In some examples, outer tubular member 502 may include a pull wire configured to allow a clinician to cause a deflection (e.g., a deflectable-curve) of a distal end of outer tubular member 502. For example, the pull wire may be actuated by sliding control button 511.

Retrieval assembly 504 may include a retrieval member 514 and a fixed-curve catheter 516. Retrieval member 514 may include an elongate member terminating in at least one snare 518. Retrieval member 514 may be controllable, e.g., by a control member 521 or other control member operable by a clinician, to slid in the proximal-distal direction or rotate. At least one snare 518 may be moveable (e.g., controllable), by a torque member 520 or other control member operable by a clinician, between an expanded configuration and a contracted configuration. Fixed-curve catheter 516 may include a resilient material defining elongate member having a lumen and terminating in distal tip 524. Fixed-curve catheter 516 may include a distal curve 522 adjacent and proximal to distal tip 524. For example, in a relaxed state, e.g., without application of external forces to fixed-curve catheter 516, distal curve 522 may include a deflection of a distal portion of fixed-curve catheter 516 relative to the longitudinal axis of retrieval assembly 504, e.g., a portion of fixed-curve catheter 516 proximal to distal curve 522. Although illustrated in FIG. 5B as a deflection of approximately 90-degrees (e.g., as indicated by deflection angle 523), distal curve 522 may include any suitable deflection angle 523, such as for example a deflection between about 1-degree and about 180-degrees, such as between about 45-degree and 135-degrees. In some examples, a local environment surrounding retrieval assembly 504 may affect deflection angle 523. For example, deflection angle 523 may be relatively greater in an ambient environment and relatively less inside the environment of a patient's body due to, for example, a temperature of the patient's body. In some examples, a proximal end 526 of fixed-curve catheter 516 may define a control member configured to manipulate fixed-curve catheter 516, such as, for example, to slid in the proximal-distal direction or rotate.

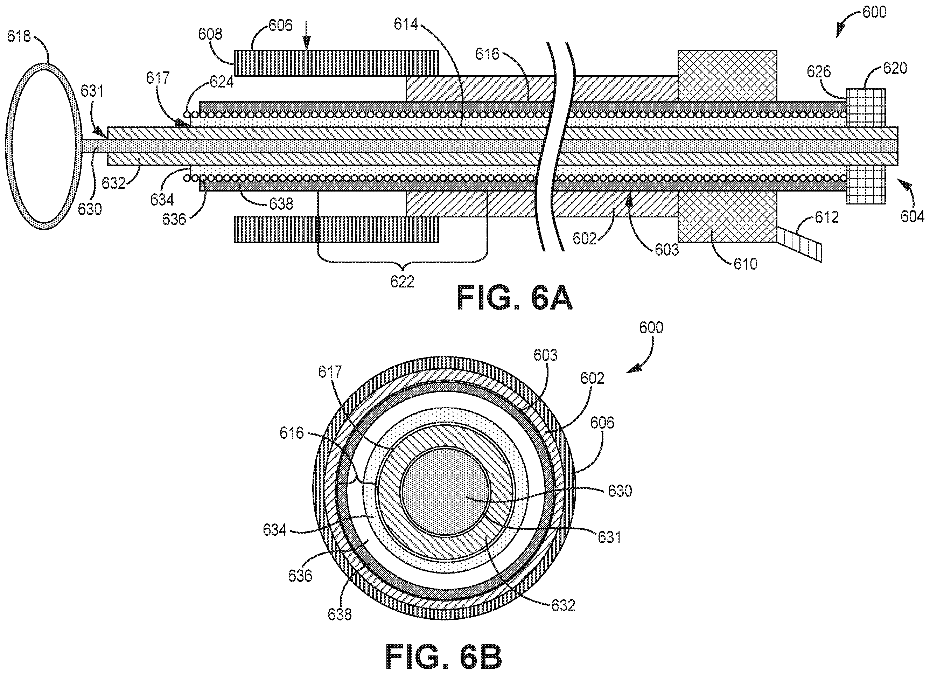

The elongate outer tubular member, fixed-curve catheter, and retrieval member may be arranged coaxially, e.g., relative to a longitudinal axis of the IMD retrieval system. FIGS. 6A and 6B are conceptual and schematic diagrams illustrating cross-sectional side and axial views of an example IMD retrieval system 600. IMD retrieval system 600 may be the same as or substantially similar to IMD retrieval system 500 discussed above in reference to FIGS. 5A-5D. For example, IMD retrieval system 600 includes an elongate outer tubular member 602 and a retrieval assembly 604.

Outer tubular member 602 may define a longitudinally extending lumen 603 terminating in a distal cup 606 having a distal opening 608. Outer tubular member 602 may include a proximal handle 610 configured to enable a clinician to manipulate outer tubular member 602, e.g., to manipulate a pull wire of . In some examples, outer tubular member 602 may include any suitable construction, such as, for example, a coiled or braided metal coated on an external surface and internal surface with one or more polymers.

Retrieval assembly 604 may include a retrieval member 614 and a fixed-curve catheter 616. Retrieval member 614 may include a retrieval member catheter 632 defining a lumen 631 within which an elongate retrieval member 630 extends in sliding engagement. Retrieval member 614 may include any suitable construction, such as, for example, a polymeric tube. Elongate retrieval member 630 terminates in at least one snare 618. Elongate retrieval member 630 may include a braided, non-braided, or partially braided wire including any suitable material, such as, for example, a metal, nickel titanium alloy, stainless steel, or the like. In some examples, at least one snare 618 may be moveable (e.g., controllable), via a torque member 620 or other control member operable by a clinician. In some examples, drawing elongate retrieval member 630 in a proximal direction into retrieval member catheter 632 may cause at least one snare 618 to convert from an expanded configuration to a contracted configuration.

In some examples, retrieval member 614 may extend in sliding engagement within the lumen of fixed-curve catheter 616. For example, fixed-curve catheter 616 may include lumen 617 configured to receive retrieval member 614 such that retrieval member 614 extends through lumen 617. Fixed-curve catheter 616 is straight for the purposes of illustrations, however, in some examples, fixed-curve catheter may include a deflection such that distal tip 624 and/or a portion of distal curve 622 may contact an interior wall of distal cup 606. In some examples, retrieval member 614 may be sufficiently flexible to conform to the shape of fixed-curve catheter 616, e.g., distal curve 622. In this way, retrieval member 614 and fixed-curve catheter 616 may be configured to define coaxial retrieval assembly 604.

In some examples, fixed-curve catheter 616 may include a multilayer construction. For example, fixed-curve catheter 616 may include core layer 636, an interior layer 634, and an exterior layer 638. Core layer 636 may include a coiled metal wire or braided metal wire. For example, core layer 636 may include a single filar or bifilar metal wire formed on a mandrel. Interior layer 634 may include any suitable polymer, such as, for example, polytetrafluoroethylene, high-density polyethylene, poly ether block amide, or another polymer configured to enable retrieval member 614 to readily slide in lumen 617. Exterior layer 638 may include any suitable polymer, such as, for example, polyethylene, polypropylene, polyether block amide or another polymer configured to enable fixed-curve catheter 616 to readily slide in lumen 603. In some examples, the polymer of exterior layer 638 may be selected to be flowable between filars of core layer 636 during formation of exterior layer to enable the catheter to retain the curved shape, e.g., during heating. In some examples, distal tip 624 of fixed-curve catheter 616 may include only core layer 636. For example, interior layer 634 and exterior layer 638 may not extend distally as far as core layer 636. In some examples, distal tip 624 may include separate metal coil wound around a distal end of fixed-curve catheter 616. In some examples, distal tip 624 may include separate over-molded polymer, such as, for example, polyether block amide with radiopaque additives, such as bismuth subcarbonate, barium sulfate, or tungsten. The separate metal coil or over-molded polymer may be more robust than a distal tip including only core layer 636. By including distal tip 624, the distal end of fixed-curve catheter 616 may be configured to engage snare 618 and/or a portion of an IMD 300 when retrieval member 614 is engaged with an IMD 300 and drawn in a proximal direction into lumen 617 of fixed-curve catheter 616.

In some examples, fixed-curve catheter 616 may include regions of increased stiffness compared to other regions of fixed-curve catheter 616. For example, a region of core layer 636 defining distal curve 622 of fixed-curve catheter 616 may include a region of increased stiffness compared to a region of fixed-curve catheter 616 proximal to distal curve 622. Stiffness of core layer 636 may be controlled by, for example, controlling a pitch of the filars of core layer 636. In some examples, stiffness of fixed-curve catheter 616 may be controlled by selection or treatment of interior layer 634 and/or exterior layer 638. For example, a region of increased stiffness may include a thicker exterior layer 638 or the region may be heat treated to stiffen the polymer of exterior layer 638. In some examples, interior layer 634 and/or exterior layer 683 may include a plurality of segments, each respective segment including one or more polymers having a selected durometer to provide a desired stiffness of the respective segment. Controlling the stiffness of fixed-curve catheter 616 may improve the ability of distal curve 622 to take on a desired curved configuration when extended from the distal cup 606 and/or increase the flexibility of fixed-curve catheter 616 proximal to distal curve 622 to better enable IMD retrieval system 600 to be introduced into the vasculature of the patient.

In some examples, fixed-curve catheter 616 may extend in sliding engagement within lumen 603 of outer tubular member 602. For example, outer tubular member 602 may include lumen 603 configured to receive fixed-curve catheter 616 such that retrieval assembly 604 extends through lumen 603. In this way, retrieval assembly 604 and outer tubular member 602 may be configured to define coaxial retrieval system 600.

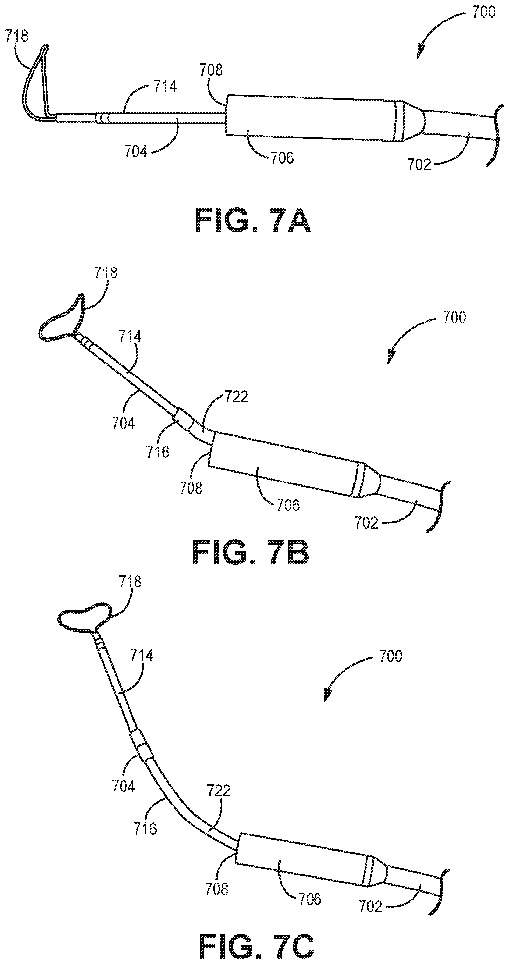

In some examples, the distal cup and the fixed-curve catheter are configured to adjustably control the degree of the distal curve, e.g., the angle between the longitudinal axis of the distal cup and the snare. FIG. 7A-7E are conceptual diagrams illustrating adjustable control of the degree of curve of the distal end of an example IMD retrieval system 700. IMD retrieval system 700 may be the same as or substantially similar to IMD retrieval systems 400, 500, and/or 600 discussed above in reference FIGS. 4A-6B. For example, IMD retrieval system 700 includes outer tubular member 702 and retrieval assembly 704 that includes retrieval member 714 and fixed-curve catheter 716. As discussed above, fixed-curve catheter 716 may extend in sliding engagement within a lumen of elongate outer tubular member 702. By including distal curve 722 in sliding engagement with the lumen of outer tubular member 702, the degree of distal curve 722 of fixed-curve catheter 716 is controllable.

As illustrated in FIG. 7A, distal curve 722 may take a substantially straight configuration when retracted into distal cup 706 of outer tubular member 702. For example, when distal curve 722 of the fixed-curve catheter remains inside the distal cup 706 (e.g., proximal distal opening 708), a longitudinal axis of retrieval member 714 may be substantially the same as the longitudinal axis of distal cup 706. In some examples, substantially the same may include an angle of the longitudinal axis of retrieval member 714 relative to longitudinal axis of distal cup 706 of less than 10-degrees, such as less than 5-degrees or less than 1-degree. In some examples, distal cup 706 is sufficiently rigid, and fixed-curve catheter is sufficiently flexible, such that distal cup 706 urges fixed-curve catheter 716 into a substantially straight configuration, e.g., straight or nearly straight relative to the longitudinal axis of distal cup 706.

As illustrated in FIG. 7B, distal curve 722 may take a partially curved configuration when partially extended from distal opening 708 of distal cup 706. For example, when distal curve 722 is at least partially extended distal to distal opening 708 of distal cup 706, a longitudinal axis of retrieval member 714 may bend at an angle relative to the longitudinal axis of distal cup 706. In some examples, the angle of the longitudinal axis of retrieval member 714 relative to longitudinal axis of distal cup 706 may be between about 1-degree and about 45-degrees, such as between about 10-degrees and about 40-degrees.

As illustrated in FIG. 7C, if more of distal curve 722 extends out of distal cup 706, the curve of fixed-curve catheter 716 may be more pronounced. For example, the angle of the longitudinal axis of retrieval member 714 relative to longitudinal axis of distal cup 706 may be between about 30-degree and about 70-degrees, such as between about 30-degrees and about 50-degrees. The adjustable curve of fixed-curve catheter 716 provides enhanced steering of the snare 718. For example, by enabling a clinician to control a degree of curvature of the fixed-curve catheter 716, the clinician may more accurately and efficiently guide snare 718 to an IMD, such as IMD 300.

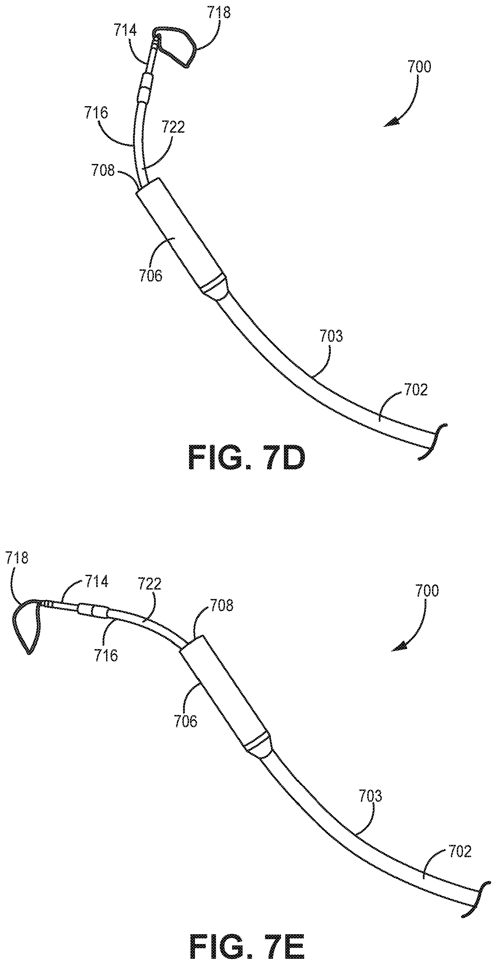

In some examples, as illustrated in FIGS. 7D and 7E, outer tubular member 702 may include a deflectable-curve portion 703. For example, deflectable-curve portion 703 may be fixed in the same manner as discussed above with respect to fixed-curve catheter 716 (e.g., fixed-curve catheter 616). In examples in which outer tubular member 702 includes deflectable-curve portion 703, fixed-curve catheter 716 may enable secondary curves of IMD retrieval system 700. The secondary curves may be manipulated by a clinician, e.g., by controlling the degree of curvature of the fixed-curve catheter, the radial orientation of the distal curve of the fixed-curve catheter relative to the curve of the elongate outer tubular member, or both, to improve control of the snare in three-dimensional space. For example, as illustrated in FIG. 7D, fixed-curve catheter 716 may be axially oriented relative to outer tubular member 702, such that distal curve 722 and deflectable-curve portion 703 generally curve in the same direction. In this way, the total deflection of snare 718 may be greater than if a retrieval device with only one of distal curve 722 or deflectable-curve portion 703. As illustrated in FIG. 7E, fixed-curve catheter 716 may be axially oriented relative to outer tubular member 702, such that distal curve 722 and deflectable-curve portion 703 generally curve in different directions. By curving in different directions, a clinician may have greater control of the position and orientation of snare 718 in a three-dimensional space. Greater control of the position and orientation of snare 718 in a three-dimensional space may reduce die time necessary for die clinician to properly receive the IMD in snare 718.

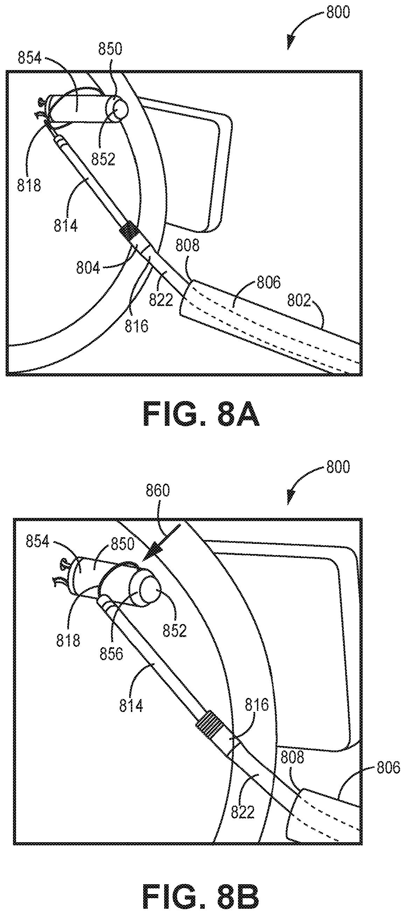

After navigating the snare of an IMD retrieval system to receive and IMD, the fixed-curve catheter may facilitate engagement of the snare with the IMD, such as a proximal retrieval feature on the IMD. FIG. 8A-8H are conceptual diagrams illustrating engagement of snare 818 of IMD retrieval system 800 with proximal retrieval feature 852 on IMD 850. IMD retrieval system 800 may be the same as or substantially similar to IMD retrieval systems 400, 500, 600, and/or 700 discussed above in reference FIGS. 4A-7E. For example, IMD retrieval system 800 includes outer tubular member 802 having distal cup 806 with distal opening 808 and retrieval assembly 804 that includes retrieval member 814 having snare 818 and fixed-curve catheter 816 having distal curve 822. As discussed above, fixed-curve catheter 816 may extend in sliding engagement within a lumen of elongate outer tubular member 802 and axially rotate around a longitudinal axis of retrieval assembly 804. By including distal curve 822 in sliding engagement with and axially rotatable relative to outer tubular member 802, the degree of distal curve 822, as well as the position and orientation of snare 818 in three-dimensional space, is controllable by a clinician.

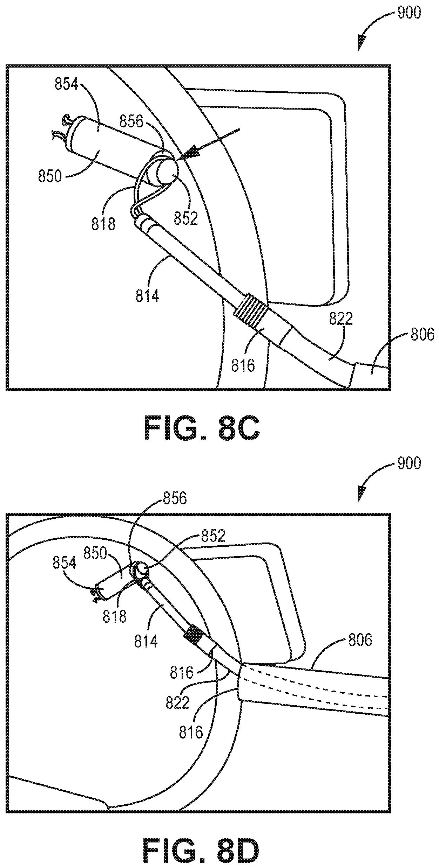

As illustrated in FIG. 8A, snare 818 may receive IMD 850 by encircling housing 854 of IMD 850. As illustrated in FIG. 8B, when snare 818 receives IMD 850 (e.g., goes over housing 854), the clinician may control the degree and/or orientation of distal curve 822 of fixed-curve catheter 816 to apply a side load (e.g., in the direction indicated by arrow 860) to housing 854. In some examples, the side load may slightly tip IMD 850. Application of a side load to the IMD housing may be urge at least a portion of snare 818 to ride along housing 854 as snare 818 is slid proximally over housing 854, the side load helps snare 818 catch groove 856 of proximal retrieval feature 852 on IMD 850, as illustrated in FIG. 8C. In some examples, snare 818 may be controlled by the clinician from an expanded configuration to a partially contracted configuration before snare 818 is slid proximally over housing 854. The partial contracted configuration, together with application of the side load, may prevent snare 818 from slipping over proximal retrieval feature 852 as snare 818 is slid proximally over housing 854. After catching groove 856, snare 818 may be controlled by the clinician from an expanded configuration to a contracted configuration (e.g., fully contracted configuration) to engage IMD 850, as illustrated in FIG. 8D.

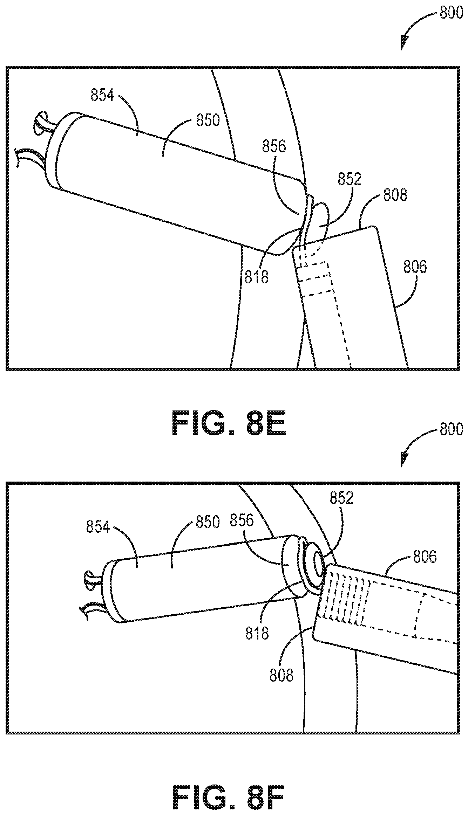

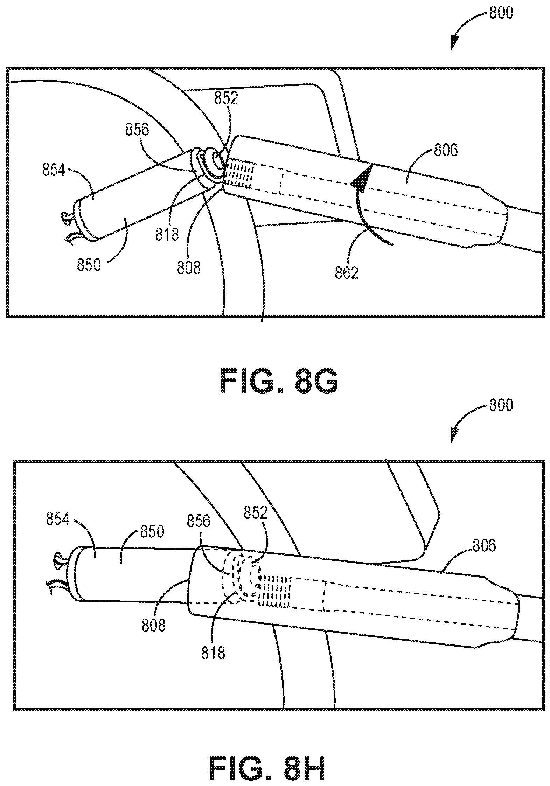

As illustrated in FIGS. 8E and 8F, in some examples, once snare 818 has engaged IMD 850, e.g., via proximal retrieval feature 852, the orientation of IMD 850 relative to distal cup 806 may make it difficult to seat distal cup 806 over IMD 850 prior to retrieval. For example, distal opening 808 of the distal cup may catch in groove 856 of proximal retrieval feature 852. As illustrated in FIG. 8G, axial rotation of outer tubular member 802 relative to fixed-curve catheter 816 engaged with IMD 850, e.g., as indicated by arrow 862, may facilitate reorienting distal cup 806 relative to IMD 850. In other examples, axial rotation of the fixed-curve catheter may enable distal cup 860 to change orientation relative to IMD 850. As illustrated in FIG. 8H, changing the orientation of distal cup 806 relative to IMD 850 may guide distal cup 806 to an orientation that allows IMD 850 to seat (e.g., be retrieved) in distal cup 806. By facilitating engagement with the IMD, the system may reduce the time to properly engage the IMD for retrieval.

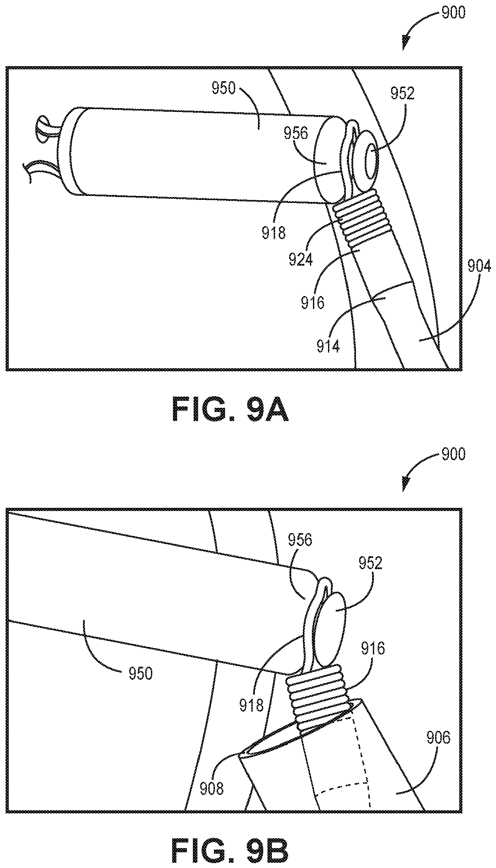

In some examples, a distal end of a fixed-curve catheter may be used to facilitate seating of an IMD in the distal cup. FIG. 9A-9F are conceptual diagrams illustrating retrieval of an implantable medical device by urging distal end 924 of fixed-curve catheter 916 against proximal retrieval feature 952 of IMD 950 to facilitate seating of IMD 950 in distal cup 906 of IMD retrieval system 900. IMD retrieval system 900 may be the same as or substantially similar to IMD retrieval systems 400, 500, 600, 700, and/or 800 discussed above in reference FIGS. 4A-3H. For example, IMD retrieval system 900 includes outer tubular member 902 having distal cup 906 with distal opening 908 and retrieval assembly 904 that includes retrieval member 914 having snare 918 and fixed-curve catheter 916 having distal curve (not shown). As discussed above, retrieval member 914 may extend in sliding engagement within a lumen of fixed-curve catheter 916. The sliding engagement may enable a clinician to advance distal end 924 of fixed-curve catheter to IMD 950 to facilitate seating of distal cup 906 onto IMD 950.

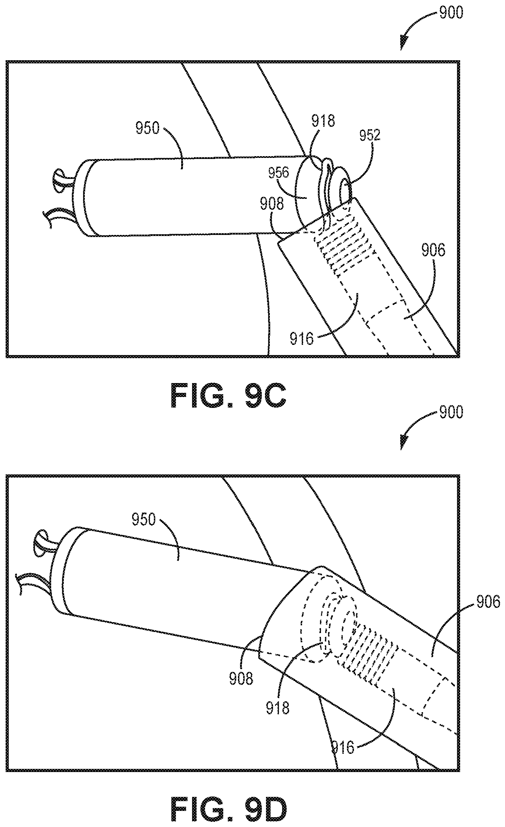

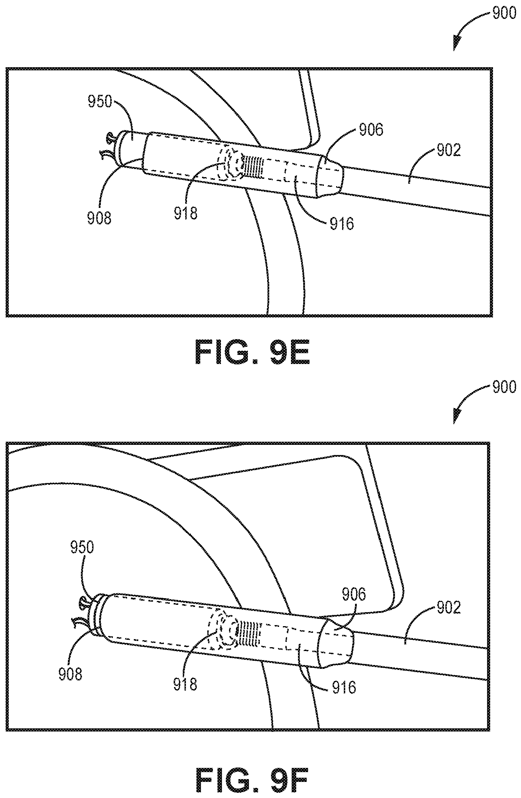

Once snare 918 has engaged IMD 950, e.g., proximal retrieval feature 952, as illustrated in FIG. 9A, distal end 924 of fixed-curve catheter 916 may be slid up to proximal retrieval feature 952, as illustrated in FIG. 9B. In some examples, as illustrated in FIG. 9C, distal end 924 may include a coil tip that is sized to divert distal cup 906 away from groove 956 of proximal retrieval feature 952. In other words, with the coil tip of the fixed-curve catheter pressed into the IMD, the groove of the proximal retrieval feature is covered, and the distal cup is prevented from catching on the groove. In this way, as illustrated in FIG. 9D, distal cup 906 may be advanced over IMD 950 without catching groove 956. Then, as illustrated in FIGS. 9E and 9F, distal cup 906 may be advanced over IMD 950 to fully seat IMD 950 within distal cup 906.

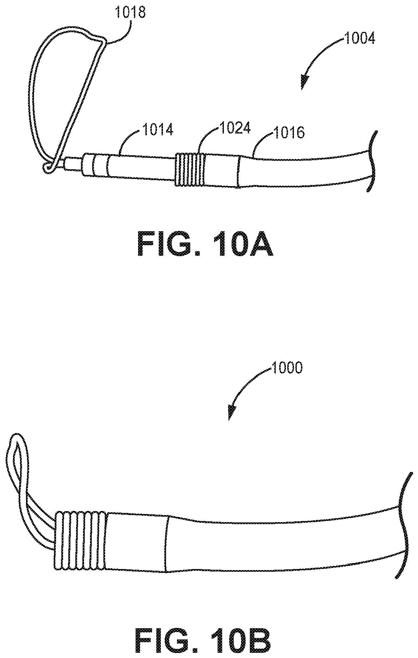

In some examples, the fixed-curved catheter may be used as part of the snare cinching mechanism. FIG. 10A and 10B are conceptual diagrams illustrating retrieval of IMD 1050 by using fixed-curved catheter 1016 of IMD retrieval system 1000 to cinch snare 1018 into a contracted configuration. IMD retrieval system 1000 may be the same as or substantially similar to IMD retrieval systems 400, 500, 600, 700, 800, and/or 900 discussed above in reference FIGS. 4A-9F. For example, IMD retrieval system 1000 includes outer tubular member (not shown) and retrieval assembly 1004 that includes retrieval member 1014 having snare 1018 and fixed-curve catheter 1016 having distal curve (not shown). As discussed above, retrieval member 1014 may extend in sliding engagement within a lumen of fixed-curve catheter 1016. Tire sliding engagement may enable a clinician to advance distal end 1024 of fixed-curve catheter 1016 to snare 1018 to facilitate controlling snare 1018 from an expanded configuration to a contracted configuration.

As illustrated in FIG. 10A, snare 1018 may include an expanded configuration. The expanded configuration includes a loop sized to receive an IMD, e.g., to fit over the housing of the IMD. As illustrated in FIG. 10B, distal end 1024 of fixed-curve catheter 1016 may be advanced in the distal direction to snare 1018. In some examples, retrieval member 1014 may be drawn in die proximal direction to effectively advance distal end 1024 to snare 1018. Advancing distal end 1024 toward snare 1018 may draw snare 1018 into the lumen of fixed-curve catheter 1016, For example, when an IMD is receive in snare 1018, advancing distal end 1024 over snare 1018 may control snare 1018 to take a contracted configuration to engage the IMD, such as the proximal retrieval feature of the IMD. In this way, advancing distal end 1024 over snare 1018 may lock snare 1018 in the contracted configuration, thereby securely engaging an IMD, which may enable a clinician to manipulate the orientation of the outer tubular member and/or fixed-curve catheter relative to the IMD without snare 1018 disengaging from the IMD.

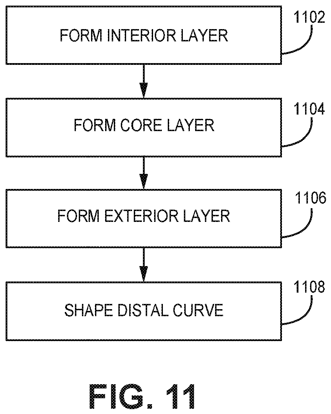

The IMD retrieval systems described herein may be manufactured using any suitable technique. FIG. 11 is a flow diagram illustrating a non-claimed example method of manufacturing a fixed-curve catheter of an IMD retrieval system. Although the technique illustrated in FIG. 11 is described in reference to IMD retrieval system 600 illustrated in reference to FIGS. 6A and 6B, the technique may be used to manufacture other IMD retrieval systems, such as IMD retrieval systems 400, 500, 700, 800, 900, and/or 1000. Additionally, IMD retrieval systems 400, 500, 700, 800, 900, and/or 1000 may be manufactured using other techniques,

The technique illustrated in FIG. 11 includes depositing a polymer to form interior layer 634 (1102). Any suitable deposition or coating method may be used to deposit interior layer 634. As discussed above, interior layer 634 may include any suitable polymer, such as, for example, polytetrafluoroethylene.

The technique illustrated in FIG. 11 includes forming core layer 636 (1104). In some examples, forming core layer 636 may include winding one or more metal wires onto a mandrel. In some examples, forming core layer 636 may include braiding two or more metal wires onto a mandrel. In some examples, forming core layer 636 may include forming core layer 636 directly over interior layer 634.

The technique illustrated in FIG. 11 includes depositing a polymer onto an exterior surface of core layer 636 to form exterior layer 638 (1106). Any suitable deposition or coating method may be used to deposit exterior layer 638. As discussed above, exterior layer 638 may include any suitable polymer, such as, for example, polyether block amide. Exterior layer 638 may at least partially flow into spaces between filars of core layer 636.

In some examples, the technique may include forming distal tip 624, e.g., by winding a metal coil or over-molding a polymer onto fixed-curve catheter 616. In some examples, the technique may include forming an over-molded proximal hub, such as, for example, onto proximal end 626.

The technique illustrated in FIG. 11 includes shaping fixed-curve catheter 616 to form distal curve 622 (1108). In some examples, shaping core layer 636 may include heating fixed-curve catheter 616, bending fixed-curve catheter 616, and cooling fixed-curve catheter 616 to set the distal curve 622. In some examples, forming core layer 636 and shaping core layer 636 may be performed in a single step. For example, forming core layer 636 may include winding (or braiding) metal wires onto a mandrel including a curve that substantially corresponds to the described curvature of distal curve 622.

In some examples, the technique may include forming outer tubular member 602. In some examples, die technique may include forming retrieval member 614. In some examples, the technique may include inserting retrieval member 614 into lumen 617 of fixed-curve catheter 616 to form retrieval assembly 604. In some examples, the technique may include inserting retrieval assembly 604into lumen 603 of outer tubular member 602 to form IMD retrieval system 600,

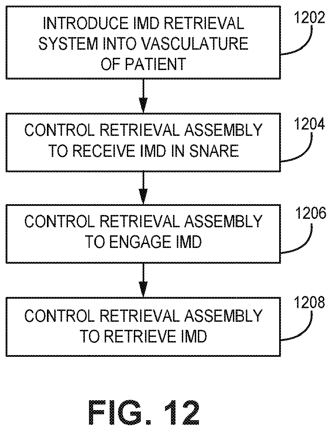

FIG. 12 is a flow diagram illustrating a non-claimed example method of using an implantable medical device retrieval system. Although the technique illustrated in FIG. 12 is described in reference to IMD retrieval system 500 illustrated in reference to FIGS. 5A-5D, the technique may be used with other IM D retrieval systems, such as IMD retrieval systems 400, 600, 700, 800, 900, and/or 1000, Additionally, IMD retrieval systems 400, 600, 700, 800, 900, and/or 1000 may be used with other techniques.

The technique illustrated in FIG. 12 includes introducing the IMD retrieval system 500 into the vasculature of a patient (1202). In some examples, introducing the IMD retrieval system 500 may include creating an incision in the femoral vein of the patient. In some examples, introducing the IMD retrieval system 500 may include navigating, with the assistance of fluoroscopy, the IMD retrieval system 500 to a target chamber of the heart of the patient. In some examples, introducing the IMD retrieval system 500 may include aligning distal tip 524 of fixed-curve catheter 516 adjacent and/or touching the IMD. In some examples, the position of the distal tip 52.4 of fixed-curve catheter 516 may be confirmed using two orthogonal views via fluoroscopy.

The technique illustrated in FIG. 12 includes controlling retrieval assembly 504 to receive the IMD in snare 518 (1204). For example, a clinician may manipulate handle 510 and/or torque member 5 20 coupled to fixed-curve member 516 and/or outer tubular member 502 to position and orient snare 518 to loop snare 518 over a housing of the IMD. In some examples, prior to tightening snare 518, the clinical may manipulate handle 510 to apply tension to snare 518 in a direction transverse to a longitudinal axis of the IMD. While applying transverse tension, the clinician may control retrieval assembly 504 to move snare 518 toward a proximal end of the IMD, e.g., toward the proximal retrieval feature, until snare 518 engages the catch groove of the proximal retrieval feature.

The technique illustrated in FIG. 12 includes controlling retrieval assembly 504 to engage the IMD (1206). For example, as discussed above, a clinician may manipulate the retrieval member 514 to control snare 518 from an expanded configuration to a contracted configuration. Additionally, or alternatively, a clinician may manipulate fixed-curve catheter to control snare 5 18 from an expanded configuration to a contracted configuration and/or to effectively lock snare 518 in the contracted configuration.

The technique illustrated in FIG. 12 includes controlling retrieval assembly 504 to retrieve the IMD (1208). For example, as discussed above, a clinician may manipulate fixed-curve member 316 and/or outer tubular member 502 to position and orient distal cup 506 to seat the IMD inside the distal cup In some examples, retrieving the IMD may include application of a proximal force sufficient to dislodge the IMD from the tissue of the patient. In some examples, retrieving the IMD may include withdrawing the IMD retrieval system from the vasculature of the patient.

Various examples of the disclosure have been described. Any combination of the described systems, operations, or functions is contemplated

## Claims

1. An implantable medical device (IMD) retrieval system comprising:
an elongate outer tubular member (502) including a longitudinally extending lumen terminating in a distal cup (506), wherein the distal cup is configured to receive the IMD; and
a retrieval assembly (504) comprising:
a retrieval member (514) comprising an elongate member terminating in at least one snare (518), wherein the at least one snare is moveable between an expanded configuration and a contracted configuration; and
a fixed-curve catheter (516) comprising a resilient material forming a distal curve and defining a lumen in which the retrieval member extends in sliding engagement, wherein the fixed-curve catheter extends in sliding engagement within the lumen of the elongate outer tubular member,
wherein the fixed-curve catheter is rotatable relative to the elongate outer tubular member.

2. The system of claim 1, wherein the fixed-curve catheter is controllable between a substantially straight configuration when retracted into the distal cup of the elongate outer tubular member and an at least partially curved configuration when extended at least partially from the distal end of the distal cup.

3. The system of claim 1 or 2, wherein the fixed-curve catheter (616) comprises:
an elongate core layer (636) comprising a coiled or braided metal wire, wherein the core layer defines an exterior surface and an interior surface defining the lumen of the fixed-curve catheter;
an inner layer (634) disposed on the interior surface of the core layer; and
an outer layer (638) disposed on the exterior surface of the core layer.

4. The system of claim 3, wherein the metal wire of the core layer comprises stainless steel.

5. The system of claim 3 or 4, wherein the inner layer comprises polytetrafluoroethylene.

6. The system of any of claims 3-5, wherein the outer layer comprises polyether block amide.

7. The system of any of claims 1-6, wherein the fixed-curve catheter further is rotatable relative to the retrieval member.

8. The system of any of claims 1-7, wherein the elongate outer tubular member includes a deflectable-curve proximal to the distal cup.

9. The system of any of claims 1-8, wherein the retrieval member comprises a loop snare (518) having at least one wire loop configured to engage the IMD in a contracted configuration of the at least one wire loop.

10. The system of any of claims 1-9, wherein the at least one snare is configured to engage a proximal retrieval feature (310) disposed on a proximal end (381) of a housing (380) of the IMD.

11. The system of any of claims 1-10, wherein the expanded configuration of the at least one snare is configured to travel over a housing (380) of the IMD, and wherein the contracted configuration of the at least one snare is configured to mechanically couple the at least one snare to a proximal retrieval feature (310) disposed on a proximal end (381) of a housing (380) of the IMD.

## Patentansprüche

1. Rückholsystem für eine implantierbare medizinische Vorrichtung (IMD), umfassend:
ein längliches äußeres röhrenförmiges Element (502), einschließlich eines sich in Längsrichtung erstreckenden Lumens, das in einem distalen Becher (506) endet, wobei der distale Becher konfiguriert ist, um die IMD aufzunehmen; und
eine Rückholanordnung (504), umfassend:
ein Rückholelement (514), umfassend ein längliches Element, das in mindestens einer Schlinge (518) endet, wobei die mindestens eine Schlinge zwischen einer erweiterten Konfiguration und einer zusammengezogenen Konfiguration bewegbar ist; und
einen Katheter (516) mit fester Krümmung, umfassend ein elastisches Material, das eine distale Krümmung ausbildet, und der ein Lumen, in den sich das Rückholelement in Gleiteingriff erstreckt, definiert, wobei sich der Katheter mit fester Krümmung in Gleiteingriff innerhalb des Lumens des länglichen äußeren röhrenförmigen Elements erstreckt,
wobei der Katheter mit fester Krümmung relativ zu dem länglichen äußeren röhrenförmigen Element drehbar ist.

2. System nach Anspruch 1, wobei der Katheter mit fester Krümmung zwischen einer im Wesentlichen geraden Konfiguration, wenn er in den distalen Becher des länglichen äußeren röhrenförmigen Elements zurückgezogen wird, und einer mindestens teilweise gekrümmten Konfiguration, wenn er von dem distalen Ende des distalen Bechers mindestens teilweise gestreckt wird, steuerbar ist.

3. System nach Anspruch 1 oder 2, wobei der Katheter (616) mit fester Krümmung umfasst:
eine längliche Kernschicht (636), umfassend einen gewickelten oder geflochtenen Metalldraht, wobei die Kernschicht eine Außenoberfläche und eine Innenoberfläche definiert, die das Lumen des Katheters mit fester Krümmung definiert;
eine innere Schicht (634), die an der Innenoberfläche der Kernschicht eingerichtet ist; und
eine äußere Schicht (638), die an der Außenoberfläche der Kernschicht eingerichtet ist.

4. System nach Anspruch 3, wobei der Metalldraht der Kernschicht Edelstahl umfasst.

5. System nach Anspruch 3 oder 4, wobei die innere Schicht Polytetrafluorethylen umfasst.

6. System nach einem der Ansprüche 3 bis 5, wobei die äußere Schicht Polyetherblockamid umfasst.

7. System nach einem der Ansprüche 1 bis 6, wobei der Katheter mit fester Krümmung ferner relativ zu dem Rückholelement drehbar ist.

8. System nach einem der Ansprüche 1 bis 7, wobei das längliche äußere röhrenförmige Element eine auslenkbare Krümmung proximal zu dem distalen Becher einschließt.

9. System nach einem der Ansprüche 1 bis 8, wobei das Rückholelement eine Schlaufenschlinge (518) umfasst, die mindestens eine Drahtschlaufe aufweist, die konfiguriert ist, um in einer zusammengezogenen Konfiguration der mindestens einen Drahtschlaufe die IMD in Eingriff zu nehmen.

10. System nach einem der Ansprüche 1 bis 9, wobei die mindestens eine Schlinge konfiguriert ist, um ein proximales Rückholmerkmal (310), das an einem proximalen Ende (381) eines Gehäuses (380) der IMD eingerichtet ist, in Eingriff zu nehmen.

11. System nach einem der Ansprüche 1 bis 10, wobei die erweiterte Konfiguration der mindestens einen Schlinge konfiguriert ist, um sich über ein Gehäuse (380) der IMD zu bewegen, und wobei die zusammengezogene Konfiguration der mindestens einen Schlinge konfiguriert ist, um die mindestens eine Schlinge mit einem proximalen Rückholmerkmal (310), das an einem proximalen Ende (381) eines Gehäuses (380) der IMD eingerichtet ist, mechanisch zu koppeln.

## Revendications

1. Système de récupération d'un dispositif médical implantable (DMI) comprenant :
un élément tubulaire extérieur allongé (502) y compris un lumen s'étendant longitudinalement se terminant par une coupelle distale (506), dans lequel la coupelle distale est configurée pour recevoir le DMI ; et
un ensemble de récupération (504) comprenant :
un élément de récupération (514) comprenant un élément allongé se terminant par au moins un collet (518), dans lequel l'au moins, un collet est déplaçable entre une configuration expansée et une configuration contractée ; et
un cathéter à courbe fixe (516) comprenant un matériau résilient formant une courbe distale et définissant un lumen dans lequel l'élément de récupération s'étend en engagement coulissant, dans lequel le cathéter à courbe fixe s'étend en engagement coulissant à l'intérieur du lumen de l'élément tubulaire externe allongé,
dans lequel le cathéter à courbe fixe est rotatif par rapport à l'élément tubulaire externe allongé.

2. Système selon la revendication 1, dans lequel le cathéter à courbe fixe est contrôlable entre une configuration sensiblement droite lorsqu'il est rétracté dans la coupelle distale de l'élément tubulaire extérieur allongé et une configuration au moins partiellement incurvée lorsqu'il est prolongé au moins partiellement à partir de l'extrémité distale de la coupelle distale.

3. Système selon la revendication 1 ou 2, dans lequel le cathéter à courbe fixe (616) comprend :
une couche de base allongée (636) comprenant un fil métallique enroulé ou tressé, dans lequel la couche de base définit une surface extérieure et une surface intérieure définissant le lumen du cathéter à courbe fixe ;
une couche intérieure (634) disposée sur la surface intérieure de la couche de base ; et
une couche extérieure (638) disposée sur la surface extérieure de la couche de base.

4. Système selon la revendication 3, dans lequel le fil métallique de la couche de base est en acier inoxydable.

5. Système selon la revendication 3 ou 4, dans lequel la couche interne est en polytétrafluoroéthylène.

6. Système selon l'une quelconque des revendications 3 à 5, dans lequel la couche externe comprend un polyéther bloc amide.

7. Système selon l'une quelconque des revendications 1 à 6, dans lequel le cathéter à courbe fixe est en outre rotatif par rapport à l'élément de récupération.

8. Système selon l'une quelconque des revendications 1 à 7, dans lequel l'élément tubulaire externe allongé comprend une courbe déflectable proximale à la coupelle distale.

9. Système selon l'une quelconque des revendications 1 à 8, dans lequel l'élément de récupération comprend un collet de boucle (518) ayant au moins une boucle de fil métallique configurée pour engager le DMI dans une configuration contractée de l'au moins une boucle de fil.

10. Système selon l'une quelconque des revendications 1 à 9, dans lequel l'au moins, un collet est configuré pour s'engager dans une caractéristique de récupération proximale (310) disposée sur une extrémité proximale (381) d'un boîtier (380) du DMI.

11. Système selon l'une quelconque des revendications 1 à 10, dans lequel la configuration étendue de l'au moins un collet est configurée pour se déplacer sur un boîtier (380) du DMI, et dans lequel la configuration contractée de l'au moins un collet est configurée pour coupler mécaniquement l'au moins un collet à une caractéristique de récupération proximale (310) disposée sur une extrémité proximale (381) d'un boîtier (380) du DMI.
